# EUROPÄISCHE PATENTANMELDUNG

(11) **EP 0 893 100 A2**
(43) Veröffentlichungstag der Anmeldung: **27.01.1999**
(21) Anmeldenummer: 98112314.4
(22) Anmeldetag: 02.07.1998
(51) Int. Cl.: A61B 17/36

(54) **Gepulste Lichtquelle**

(30) Priorität: 23.07.1997 DE 19731699
(71) Anmelder: KALTENBACH & VOIGT GmbH & Co., 88400 Biberach/Riss (DE)
(72) Erfinder: Hack, Alexander, 88400 Biberach / Riss (DE)
(74) Vertreter: Schmidt-Evers, Jürgen, Dipl.-Ing.

(57) **Zusammenfassung**

Gepulste Lichtquelle (1) zum Abtragen von biologischem Gewebe. Die gepulste Lichtquelle (1) umfaßt Lichtmittel (3) sowie eine Steuereinheit (2), die die Lichtmittel (3) derart steuert, daß die Lichtmittel einerseits Ablationsimpulse (B_{A}) mit einer zur Ablation von Gewebe ausreichenden Bestrahlungsstärke sowie andererseits eine Koagulationsstrahlung (B_{K}) mit einer lediglich für eine Erwärmung von Gewebe, jedoch nicht für eine Ablation von Gewebe ausreichenden Bestrahlungsstärke erzeugen. Die Steuereinheit (2) steuert die Lichtmittel (3) derart, daß die Lichtmittel (3) die Koagulationsstrahlung (B_{K}) zeitlich unabhängig von den Ablationsimpulsen (B_{A}) erzeugen.

## Beschreibung

Die vorliegende Erfindung betrifft eine gepulste Lichtquelle zum Abtragen von biologischem Gewebe nach dem Oberbegriff des Anspruches 1. Insbesondere betrifft die vorliegende Erfindung eine gepulste Lichtquelle der zuvor beschriebene Art zum Einsatz auf dem zahnmedizinischen Gebiet.

Bekannterweise kann mit Hilfe energiereicher Lichtstrahlung, wie z.B. Laserstrahlung, biologisches Gewebe abgetragen werden. Durch die Verwendung eines hinreichend intensiven Lichts wird jedoch das umliegende Gewebe erwärmt, wobei das Ausmaß dieser Erwärmung insbesondere von der Wellenlänge der verwendeten Strahlung bzw. den hiervon abhängigen Absorptionskoeffizienten des Gewebes sowie der Bestrahlungsstärke abhängig ist. Beim Abtragen bzw. Schneiden von biologischem Gewebe ist die dadurch hervorgerufene Vertiefung bzw. der Schnitt von einer Karbonisationszone, einer durch Vakuolen aufgelockerten Zone, einer Koagulationszone sowie einem reversibel thermisch geschädigten Bereich umgeben. Zur Erzielung eines möglichst guten Wundheilungsverlaufs sowie einer möglichst geringen Schädigung des Gewebes sind möglichst geringe thermische Wirkungen vorteilhaft. Die Ausbildung einer Karbonisationsschicht, d.h. die Karbonisation der Gewebeoberfläche, wie sie beim Schneiden mit Dauerstrichlasern auftritt, ist ungewünscht. Die durch die Erwärmung erzeugte Koagulationszone und die damit verbundene Hämostase ist jedoch dann vorteilhaft, wenn bei der Behandlung des Gewebes die Kapillarschicht beeinträchtigt wird, da ansonsten das austretende Blut die Abtragung des Gewebes beeinträchtigen würde. Die durch die Erwärmung erzeugte Koagulationszone ermöglicht somit nichtblutende Schnitte.

Es ist bekannt, daß mit gepulsten Lichtquellen hoher Leistung und einer Wellenlänge im ultravioletten oder infraroten Bereich Gewebe ohne Karbonisation der Gewebeoberfläche mit relativ geringen thermischen Schäden abgetragen werden kann. Dabei wird eine relativ geringe Koagulationszone ausgebildet, die beispielsweise eine Dicke von lediglich 30-40 µm aufweist. Eine derartig geringe Koagulationszone ist insbesondere für die Behandlung oberflächlicher Hautläsionen sowie für die kosmetische Chirurgie vorteilhaft, da eine über die Abtragung des Gewebes hinausgehende Schädigung des Gewebes weitgehend vermieden wird. Wie jedoch bereits zuvor beschrieben worden ist, kann mit einer derartig geringen Koagulationszone bei Beeinträchtigung der Kapillarschicht des Gewebes kein nichtblutender Schnitt erzielt werden, da dann das austretende Blut die Abtragung des Gewebes beeinträchtigt und ggf. sogar verhindert.

Es bestand daher das Bedürfnis nach einer Lichtquelle zum Abtragen von biologischem Gewebe, mit deren Hilfe einerseits Gewebe möglichst präzise und mit möglichst geringen thermischen Nebenwirkungen sowie ohne Karbonisation der Gewebeoberfläche abgetragen und andererseits eine für den jeweiligen Einsatzzweck spezifische Koagulationszone erzeugt und ausgebildet werden kann. Zu diesem Zweck wurde in der DE-C1-195 21 003 eine gepulste Lichtquelle gemäß dem Oberbegriff des Anspruches 1 vorgeschlagen. Diese gepulste Lichtquelle weist eine Steuereinheit auf, die die Lichtquelle derart steuert, daß die Lichtquelle mit einer vorgegebenen Impulsfrequenz kurze Ablationsimpulse zur Ablation, d.h. zum Abtragen des Gewebes, erzeugt, wobei jedem Ablationsimpuls eine Koagulationsbestrahlung folgt, deren Bestrahlungsstärke nicht für das Abtragen von Gewebe ausreicht, jedoch zu einer Erwärmung des Gewebes führt, welche wiederum die Ausbildung einer Koagulationszone unterstützt. Durch die Erzeugung der Ablationsimpulse kann somit einerseits ohne Ausbildung einer Karbonisationsschicht ein sehr effektiver thermomechanischer Ablationsprozeß mit relativ geringer thermischer Beeinträchtigung des verbleibenden Gewebes erzielt werden. Dies ist insbesondere für die Behandlung oberflächlicher Hautläsionen oder für die kosmetische Chirurgie von besonderem Interesse. Zum anderen wird mit der künstlichen Erwärmung des Gewebes durch die jedem Ablationsimpuls nachfolgende Koagulationsbestrahlung die Ausbildung einer Koagulationszone unterstützt, wobei die damit verbundene Hämostase auch bei Beeinträchtigung der Kapillarschicht des Gewebes nichtblutende Schnitte ermöglicht, d.h. selbst bei Beeinträchtigung der Kapillarschicht des Gewebes wird die Abtragung nicht durch austretendes Blut beeinträchtigt.

Gemäß der DE-C1-195 21 003 folgt jedem Ablationsimpuls stets eine Koagulationsstrahlung, wobei die Koagulationsstrahlung ebenfalls in Form von Impulsen realisiert sein kann. Die Erzeugung der Koagulationsstrahlung ist jedoch in jedem Fall von der Erzeugung eines vorhergehende Ablationsimpulses abhängig. Dies macht die Steuerung der gepulsten Lichtquelle durch die Steuereinheit aufwendig.

Der vorliegenden Erfindung liegt daher die Aufgabe zugrunde, eine gepulste Lichtquelle der vorbeschriebenen Art derart weiterzubilden, daß die Steuerung der Lichtquelle durch die Steuereinheit erleichtert wird.

Insbesondere soll auch mit der gepulsten Lichtquelle der vorliegenden Erfindung einerseits biologisches Gewebe möglichst präzise und mit möglichst geringen thermischen Nebenwirkungen sowie ohne Karbonisierung der Gewebeoberfläche abgetragen werden können und andererseits das Gewebe gezielt und steuerbar zur Ausbildung einer für den Einsatzzweck spezifischen Koagulationszone erwärmbar sein.

Die zuvor genannte Aufgabe wird gemäß der vorliegenden Erfindung durch eine gepulste Lichtquelle nach dem Anspruch 1 gelöst.

Vorteilhafte Ausgestaltungen und Weiterbildungen der vorliegenden Erfindung ergeben sich aus den Unteransprüchen.

Die erfindungsgemäße gepulste Lichtquelle erzeugt wie die aus der DE-C1-195 21 003 bekannte gepulste Lichtquelle Ablationsimpulse mit einer bestimmten Impulsfrequenz und einer zur Ablation, d.h. zum Abtragen von Gewebe, ausreichenden Bestrahlungsstärke. Des weiteren wird eine Koagulationsstrahlung erzeugt, die lediglich zu einer Erwärmung des Gewebes führt, deren Bestrahlungsstärke jedoch nicht zur Ablation von Gewebe ausreicht. Im Gegensatz zu der DE-C1-195 21 003 ist jedoch gemäß der vorliegenden Erfindung die Erzeugung der Koagulationsstrahlung nicht an das Auftreten eines vorhergehenden Ablationsimpulses gebunden. Vielmehr wird die Koagulationsstrahlung unabhängig von den Ablationsimpulsen erzeugt. Dadurch wird die Funktion der Steuereinheit zur Steuerung der gepulsten Lichtquelle erleichtert, wobei jedoch weiterhin die Vorteile der aus der DE-C1-195 21 003 bekannten gepulsten Lichtquelle beibehalten werden. D.h. durch die Erzeugung von Ablationsimpulsen mit einer zur Ablation von biologischem Gewebe ausreichenden Bestrahlungsstärke kann ohne Karbonisation ein sehr effektiver thermomechanischer Ablationsprozeß mit relativ geringer thermischer Schädigung des verbleibenden Gewebes erzielt werden. Dies ist insbesondere für die Behandlung oberflächlicher Hautläsionen sowie für die kosmetische Chirurgie von Vorteil. Durch die Erzeugung einer Koagulationsstrahlung, die keine für eine Ablation von Gewebe ausreichende Bestrahlungsstärke aufweist, kann das Gewebe künstlich erwärmt werden, so daß die Ausbildung einer Koagulationszone unterstützt wird. Dies bedeutet, daß selbst bei Beeinträchtigung der Kapillarschicht des Gewebes durch die mit der Koagulation verbundene Hämostase eine Beeinträchtigung der Gewebeabtragung bzw. des Gewebeschnitts durch austretendes Blut vermieden wird. Die Wärmenebenwirkung sowie die damit verbundene Dicke der Koagulationszone kann dem individuellen Eingriff angepaßt werden, so daß sich in jedem Fall eine gerade ausreichende und somit minimal schädigende thermische Nekrosezone erzeugen läßt. Die künstlich vergrößerte Koagulationszone beeinträchtigt zudem nicht die Schnittqualität, so daß sich mit Hilfe der erfindungsgemäßen gepulsten Lichtquelle in jedem Fall nichtblutende Schnitt mit größtmöglicher Präzision realisieren lassen.

Die Ablationsimpulse sowie die Koagulationsstrahlung können sowohl von ein und denselben Lichtmitteln als auch von zwei getrennten Lichtmitteln erzeugt werden. Gemäß einem bevorzugten Ausführungsbeispiel weist jedoch die gepulste Lichtquelle lediglich ein Lichtmittel auf, wie z.B. einen Er:YAG-Laser mit einer Wellenlänge im infraroten Bereich, das von der Steuereinheit der gepulsten Lichtquelle derart angesteuert wird, daß das Lichtmittel sowohl die Ablationsimpulse als auch die Koagulationsstrahlung erzeugt. Als Lichtmittel können auch andere übliche gepulste Laserarten, wie z.B. Ho:YAG-, CO₂-, Er:YSGG-, Tm:YAG-, CO- oder Excimer-Laser, eingesetzt werden. Des weiteren ist anstelle der Verwendung von gepulsten Lasern auch der Einsatz von gepulsten Hochdruck-Gasentladungslampen, Laserdioden oder sonstigen Blitzlampen denkbar.

Gemäß einem ersten Ausführungsbeispiel der vorliegenden Erfindung wird die Koagulationsstrahlung kontinuierlich erzeugt, so daß die Ablationsimpulse der Koagulationsstrahlung überlagert werden. Dieses Ausführungsbeispiel läßt sich insbesondere leicht mit einem einzigen gepulsten Lichtmittel realisieren, welches dauerhaft die Koagulationsstrahlung erzeugt und andererseits impulsartig die Ablationsstrahlung zum Abtragen des biologischen Gewebes emittiert. Die Koagulationsstrahlung kann dabei eine zeitlich konstante Bestrahlungsstärke aufweisen. Um eine übermäßige Erwärmung des Gewebes zu vermeiden, kann jedoch auch die Koagulationsstrahlung eine sich zeitlich variierende Bestrahlungsstärke aufweisen. Diese Ausgestaltung ist auch im Hinblick auf die Tatsache sinnvoll, daß mit zunehmender Bestrahlung und zunehmender Vergrößerung des Schnittes des behandelten Gewebes der Schwellenwert der Bestrahlungsstärke, bei dem gerade noch keine Ablation des Gewebes auftritt, abnimmt. Dieser Tatsache kann mit einer zeitlich abnehmenden Bestrahlungsstärke der Koagulationsstrahlung Rechnung getragen werden. Des weiteren ist es auch möglich, eine Koagulationsstrahlung mit einer sich wellen-, sinus-, zick-zack- oder sägezahnartig usw. verändernden Bestrahlungsstärke zu erzeugen.

Gemäß einem zweiten Ausführungsbeispiel der vorliegenden Erfindung wird neben der Ablationsstrahlung auch die Koagulationsstrahlung in Form von Impulsen erzeugt. Die Koagulationsimpulse besitzen jedoch stets eine Bestrahlungsstärke, die lediglich zur Erwärmung des Gewebes ausreicht, jedoch nicht zu einer Ablation des Gewebes führen kann. Diese Koagulationsimpule werden den zum Abtragen von biologischen Gewebe dienenden Ablationsimpulsen überlagert. Da die Koagulationsstrahlung, d.h. die Koagulationsimpulse unabhängig von dem Auftreten der Ablationsimpulse erzeugt werden, kann es zu einer Phasenverschiebung zwischen den Koagulationsimpulsen und den Ablationsimpulsen kommen. Ebenso kann der Fall auftreten, daß ein Ablationsimpuls zumindest teilweise gleichzeitig mit einem Koagulationsimpuls erzeugt wird. Die Koagulationsimpulse können eine identische Bestrahlungsstärke sowie eine identische Impulsdauer aufweisen. Ebenso ist jedoch auch denkbar, daß lediglich der Energiegehalt eines jeden Koagulationsimpulses konstant gehalten wird, so daß in diesem Fall die Koagulationsimpulse unterschiedliche Bestrahlungsstärken und unterschiedliche Impulsdauern aufweisen können, wobei jedoch das Produkt aus Bestrahlungsstärke und Impulsdauer konstant ist.

Um eine bestmögliche Anpassung der Behandlung an das jeweils zu behandelnde Gewebe zu erzielen, können vorteilhafterweise die Strahlungsparameter sowohl der Ablationsimpulse als auch der Koagulationsstrahlung an den jeweiligen Anwendungsfall angepaßt und eingestellt werden. Auf diese Weise kann die Impulsdauer, die Impulsfrequenz sowie die Bestrahlungsstärke der Ablationsimpulse eingestellt werden. Des weiteren kann die Bestrahlungsstärke sowie im Falle einer impulsartigen Koagulationsstrahlung auch die Impulsdauer und die Impulsfrequenz der Koagulationsstrahlung eingestellt werden, um eine für den jeweiligen Einsatzzweck spezifische Koagulationszone, d.h. eine gerade ausreichende und somit minimal schädigende thermische Nekrosezone, ausbilden zu können.

Die vorliegende Erfindung wird nachfolgend anhand bevorzugter Ausführungsbeispiele unter Bezugnahme auf die Zeichnung näher erläutert.
Fig. 1 zeigt eine erste Variante des internen Aufbaus einer erfindungsgemäßen gepulsten Lichtquelle,
Fig. 2 zeigt eine zweite Variante des internen Aufbaus einer erfindungsgemäßen gepulsten Lichtquelle,
Fig. 3a-3c zeigen Verläufe der Ablationsstrahlung sowie der Koagulationsstrahlung gemäß einem ersten Ausführungsbeispiel der vorliegenden Erfindung,
Fig. 4a,b und Fig. 5a, 5b zeigen Varianten des in Fig. 3 dargestellten ersten Ausführungsbeispiels, und
Fig. 6a-6c zeigen Verläufe der Ablationsstrahlung sowie der Koagulationsstrahlung gemäß einem zweiten Ausführungsbeispiel der vorliegenden Erfindung, und
Fig. 7 zeigt eine mögliche Realisierung der in Fig. 2 gezeigten Variante, wobei die Koagulationsstrahlung in Übereinstimmung mit Fig. 3 erzeugt wird.

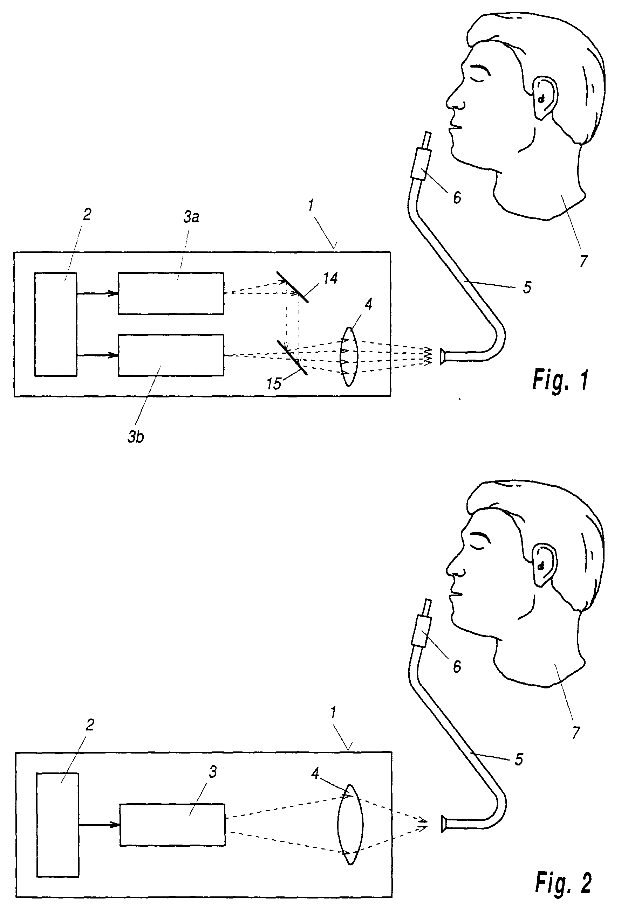

Fig. 1 zeigt den möglichen Aufbau einer erfindungsgemäßen gepulsten Lichtquelle 1. Die gepulste Lichtquelle 1 umfaßt dabei eine Steuereinheit 2, zwei getrennte Lichtmittel 3a und 3b sowie ein aus zwei Ablenkspiegeln 14,15 und einer Linsenanordnung 4 bestehendes optisches System. Die Steuereinheit 2 steuert die Lichtmittel 3a und 3b derart, daß das Lichtmittel 3a Ablationsimpulse mit einer vorgegebenen Impulsfrequenz sowie einer vorgegebenen Impulsdauer und Bestrahlungsstärke bzw. das Lichtmittel 3b eine Koagulationsstrahlung mit einer vorgegebenen Bestrahlungsstärke erzeugt. Die Bestrahlungsstärke der Ablationsimpulse ist dabei groß genug, um ein Abtragen von bioiogischem Gewebe zu erzielen. Die Bestrahlungsstärke der Koagulationsstrahlung reicht hingegen nicht zum Abtragen von Gewebe aus. Mit der Koagulationsstrahlung wird lediglich eine Erwärmung des Gewebes erzielt, um die Ausbildung einer Koagulationszone herbeizuführen. Die Ablationsstrahlung des Lichtmittels 3a sowie die Koagulationsstrahlung des Lichtmittels 3b werden über die Anlenkspiegel 14 und 15 kombiniert und mit Hilfe der Linsenanordnung 4 in einen Lichtleiter 5 eingekoppelt, an dessen anderen Ende ein Handstück 6 mit einer Lichtsonde angeordnet ist. Der Lichtleiter 5 kann mehrere einzelne Lichtleitfasern aufweisen. Mit Hilfe des Handstücks 6 und der daran befestigten Lichtsonde kann das von der gepulsten Lichtquelle 1 gelieferte Licht auf das Gewebe eines Patienten 7 zur Behandlung des Gewebes gerichtet werden.

Fig. 2 zeigt eine bevorzugte Variante der in Fig. 1 dargestellten gepulsten Lichtquelle 1. Dabei ist anstelle von zwei getrennten Lichtmitteln 3a und 3b lediglich ein gepulstes Lichtmittel 3 vorgesehen, welches sowohl die Ablationsimpulse als auch die zuvor beschriebene Koagulationsstrahlung erzeugt. Der übrige Aufbau der Lichtquelle 1 entspricht dem in Fig. 1 dargestellten Aufbau. Dadurch vereinfacht sich der interne Aufbau der gepulsten Lichtquelle 1.

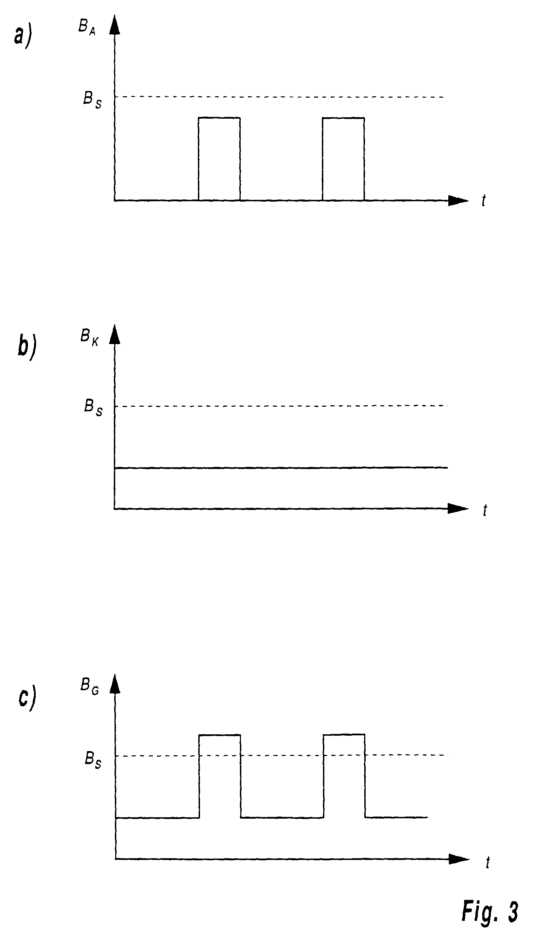

Fig. 3 zeigt beispielhaft mögliche zeitliche Verläufe der Ablationsstrahlung B_{A}, der Koagulationsstrahlung B_{K} sowie der sich daraus ergebenden Gesamtstrahlung B_{G} gemäß einem ersten Ausführungsbeispiel der vorliegenden Erfindung.

Fig. 3a zeigt dabei den impulsartigen Verlauf der Ablationsstrahlung, während Fig. 3b die kontinuierliche Erzeugung einer Koagulationsstrahlung B_{K} darstellt. Wie aus Fig. 3b hervorgeht, ist die Bestrahlungsstärke der Koagulationsstrahlung nicht hoch genug, um eine ab einem Schwellengrad B_{S} auftretende Ablation des Gewebes herbeizuführen. Durch die Überlagerung der in Fig. 3a dargestellten Ablationsstrahlung B_{A} mit der in Fig. 3b gezeigten Koagulationsstrahlung B_{K} ergibt sich der in Fig. 3c gezeigte Verlauf der Gesamtstrahlung B_{G}, wobei Fig. 3c zu entnehmen ist, daß nunmehr die durch die Überlagerung der Ablationsstrahlung B_{A} und der kontinuierlichen und konstanten Koagulationsstrahlung B_{K} ergebenden Ablationsimpulse eine Bestrahlungsstärke aufweisen, die den Schwellenwert B_{S} übersteigen, so daß die Ablationsimpulse zum Abtragen von biologischem Gewebe eingesetzt werden können. Bei dem in Fig. 3 gezeigten Ausführungsbeispiel ist es wesentlich, daß lediglich die sich durch die Addition der Ablationsstrahlung B_{A} und der Koagulationsstrahlung B_{K} ergebende Ablationsimpuse den Ablationsschwellenwert B_{S} übersteigen. Dies bedeutet, daß ggf. - wie in Fig. 3a gezeigt ist - die Bestrahlungsstärke der eigentlichen Ablationsstrahlung B_{A} aufgrund der nachfolgenden Überlagerung mit der Koagulationsstrahlung B_{K} unterhalb des Ablationsschwellenwertes B_{S} liegen kann. In der Regel wird jedoch bereits die in Fig. 3a gezeigte Ablationsstrahlung den Ablationsschwellenwert B_{S} übersteigen.

Wie in der DE-C1-195 21 003 ausführlich dargelegt ist, tritt eine Gewebeablation, d.h. ein Gewebeabtrag ein, wenn an der Gewebeoberfläche eine bestimmte, vom Gewebetyp und von der Bestrahlungsstärke abhängige Energie pro Volumenelement akkumuliert ist. Neben dieser Energie pro Volumenelement hängt der Ablationsschwellenwert B_{S} demnach auch von dem Absorptionskoeffizienten sowie der thermischen Relaxationszeit des zu behandelnden Gewebes und der Bestrahlungsstärke ab. Für einen Er:YAG-Laser kann beispielsweise von einem Ablationsschwellenwert B_{S} von 1 Jcm⁻² ausgegangen werden. Die in Fig. 3c dargestellten Ablationsimpulse müssen demnach eine Bestrahlungsstärke B_{G} > 1Jcm⁻² aufweisen. Für die in Fig. 3c gezeigten Ablationsimpulse sind beispielsweise folgende Bestrahlungsparameterbereiche möglich: Bestrahlungsstärke = 1-250 Jcm⁻², Impulsfrequenz = 1-30 Hz, Impulsdauer = 100-800 µs. Für den Fall, daß die Ablationsimpulse und die Koagulationsstrahlung mit getrennten Lichtmitteln erzeugt werden (vgl. Fig. 1), kann für die Erzeugung der Ablationsimpulse eine gepulste Lichtquelle mit einer Impulsleistung > 500 W verwendet werden.

Die Bestrahlungsstärke der Koagulationsstrahlung B_{K} liegt deutlich unterhalb des Ablationsschwellenwertes B_{S} und ruft lediglich eine Erwärmung des behandelten Gewebes hervor. Für den Fall, daß - wie in Fig. 1 gezeigt ist - die Ablationsimpulse sowie die Koagulationsstrahlung durch zwei getrennte Lichtmittel erzeugt werden, kann zur Erzeugung der Koagulationsstrahlung B_{K} eine Lichtquelle mit einer Lichtleistung beispielsweise im Bereich zwischen 0,25 und 10 W verwendet werden.

Wie aus Fig. 3 hervorgeht, wird auch für den Fall, daß die Ablationsimpulse und die Koagulationsstrahlung durch ein und dasselbe Lichtmittel erzeugt werden (vgl. Fig. 2), die Koagulationsstrahlung B_{K} stets unabhängig von den eigentlichen Ablationsimpulsen B_{A} erzeugt, d.h. das Auftreten der Koagulationsstrahlung B_{K} ist nicht von dem Auftreten eines Ablationsimpulses abhängig. Gemäß dem ersten Ausführungsbeispiel der vorliegenden Erfindung wird die Koagulationsstrahlung B_{K} dauerhaft erzeugt, wobei gemäß Fig. 3 die Bestrahlungsstärke der Koagulationsstrahlung B_{K} zeitlich konstant ist.

Im Gegensatz zu dem in Fig. 3 dargestellten zeitlichen Verlauf der Koagulationsstrahlung B_{K} ist jedoch auch ein sich zeitlich verändernder Verlauf der Bestrahlungsstärke der Koagulationsstrahlung B_{K} denkbar. Mit zunehmender Vergrößerung der Behandlungsstelle bzw. des erwärmten Bereiches des behandelten Gewebes nimmt der Ablationsschwellenwert B_{S} ab. Daher kann beispielsweise eine Koagulationsstrahlung B_{K} mit sich stetig verringernder Bestrahlungsstärke sinnvoll sein um stets sicherzustellen, daß die Bestrahlungsstärke der Koagulationsstrahlung nicht den Ablationsschwellenwert B_{S} übersteigt. Dabei ist jedoch drauf zu achten, daß zu jedem Zeitpunkt die Koagulationsstrahlung das Gewebe derart erwärmt, daß noch eine ausreichend große Koagulationszone ausgebildet werden kann.

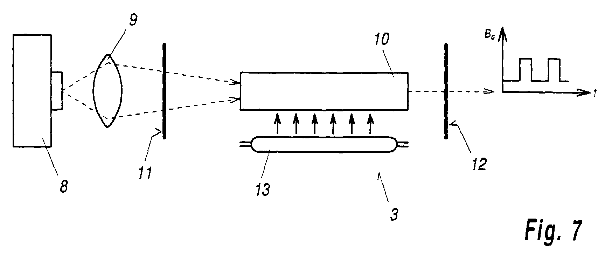

Fig. 7 zeigt eine mögliche Realisierung der in Fig. 3 gezeigten Koagulationsstrahlung mit überlagerten Ablationsimpulsen, wobei in Übereinstimmung mit der in Fig. 2 gezeigten Variante lediglich ein Lichtmittel sowohl zur Erzeugung der Koagulationsstrahlung als auch der Ablationsstrahlung eingesetzt wird.

Dabei ist gemäß Fig. 7 als Lichtmittel ein Er : YAG-Laserkristallstab 10 eingesetzt, der zur Erzeugung der Koagulationsstrahlung (vgl. Fig. 3b) kontinuierlich von einer axial anregenden Laserdiodenanordnung 8 angeregt wird, wobei die Anregungsstrahlung der Laserdiodenanordnung über eine Kollimator-Linsenanordnung 9 dem Er : YAG-Laserkristallstab 10 zugeführt wird. Der Er : YAG-Laserstab 10 ist auf bekannte Art und Weise zwischen zwei Resonatorspiegeln 11 und 12 angeordnet, um auf diese Weise eine optische Resonanz zu erzielen. Gleichzeitig wird der Er : YAG-Laserstab 10 durch eine Blitzlampe 13 gepulst betrieben wodurch die Ablationsimpulse erzeugt werden, die der Koagulationsstrahlung überlagert und schließlich als modulierte Gesamtstrahlung B_{G} von dem Lichtmittel 3 ausgegeben wird (vgl. Fig. 3a und 3c).

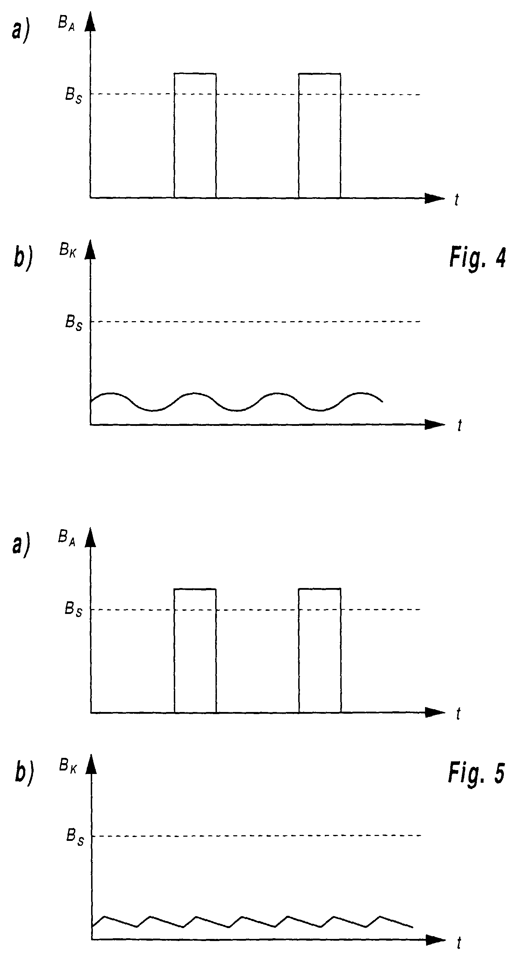

Wie in Fig. 4 und 5 dargestellt ist, kann sich die Bestrahlungsstärke B_{K} auch periodisch zeitlich verändern. Dabei ist gemäß Fig. 4b beispielsweise ein sinus- oder wellenartiger Verlauf der Bestrahlungsstärke der Koagulationsstrahlung B_{K} möglich, um eine übermäßig hohe Erwärmung des Behandlungsgewebes zu vermeiden. Gemäß Fig. 5b kann der Verlauf der Koagulationsstrahlung B_{K} auch zick-zack- oder sägezahnartig ausgebildet sein. Eine übermäßige Erwärmung der behandelten Gewebestelle durch die Koagulationsstrahlung wird auf diese Weise vermieden. Wie aus Fig. 4a bzw. Fig. 5a hervorgeht, übersteigen die jeweils dargestellten Impulse den Ablationsschwellenwert B_{S}.

Es wurde jedoch bereits anhand Fig. 3 erläutert, daß es im Grunde ausreichend ist, wenn die sich aus der Überlagerung der Impulse B_{A} mit der Koagulationsstrahlung B_{K} ergebenden Ablationsimpulse den Ablationsschwellenwert B_{S} übersteigen.

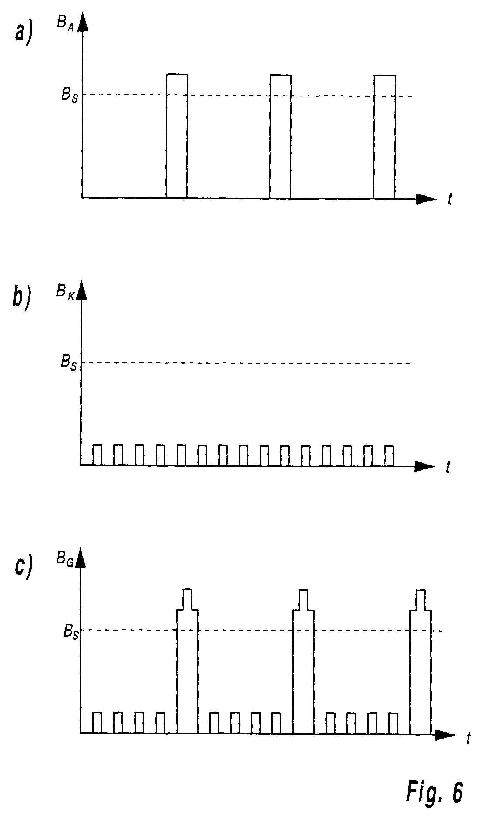

Nachfolgend soll anhand Fig. 6 ein zweites Ausführungsbeispiel der Steuerung der erfindungsgemäßen impulsten Lichtquelle erläutert werden. Dabei zeigt Fig. 6a wiederum die impulsartige Ablationsstrahlung B_{A}. Im Gegensatz zu der anhand Fig. 3-5 erläuterten Koagulationsstrahlung wird gemäß Fig. 6 die Koagulationsstrahlung nicht kontinuierlich, sondern impulsartig erzeugt. Die Koagulationsstrahlung B_{K} weist daher den in Fig. 6b gezeigten zeitlichen Verlauf auf, wobei die Bestrahlungsstärke der einzelnen Koagulationsimpulse deutlich unterhalb des Ablationsschwellenwertes B_{S} liegt. Fig. 6c zeigt den Verlauf der Gesamtstrahlung, die sich aufgrund der Überlagerung der Ablationsstrahlung B_{A} mit der Koagulationsstrahlung B_{K} ergibt. Da die Koagulationsimpulse teilweise die Ablationsimpulse überlagern, kommt es - wie Fig. 6c zu entnehmen ist - stellenweise zu einer Überhöhung der Ablationsimpulse. Bei dem in Fig. 6 gezeigten Ausführungsbeispiel genügt es, wenn der Energiegehalt jedes Koagulationsimpulses unter dem Ablationsschwellenwert liegt. Dies bedeutet, daß im Prinzip die einzelnen Koagulationsimpulse auch unterschiedliche Bestrahlungsstärken und Impulsdauern aufweisen können, wobei lediglich das Produkt aus der Bestrahlungsstärke und der Impulsdauer eines jeden Koagulationsimpulses unterhalb des entsprechenden Ablationsschwellenwertes liegen muß. Die Frequenz der Koagulationsimpulse ist deutlich höher als die der Ablationsimpulse und kann beispielsweise im Bereich um 100 Hz liegen. Entsprechend ist die Impulsdauer der Koagulationsimpulse deutlich geringer als diejenige der Ablationsimpulse und kann beispielsweise im Bereich um 10 µs liegen. Um die Wärmeentwicklung und somit die Dicke der Koagulationszone infolge der Koagulationsstrahlung an die individuellen Bedürfnisse anpassen zu können, kann die Bestrahlungsstärke, die Impulsdauer sowie die Impulsfrequenz der Koagulationsimpulse benutzerspezifisch eingestellt werden.

Auch bei dem in Fig. 6 gezeigte Ausführungsbeispiel wird die Koagulationsstrahlung, d.h. die Koagulationsimpulse, stets unabhängig von den Ablationsimpulsen erzeugt, d.h. das Auftreten der Koagulationsimpulse ist nicht an ein vorhergehendes Auftreten eines Ablationsimpulses gebunden, sondern die Koagulationsimpulse werden kontinuierlich erzeugt. Auf diese Weise kann die Steuerung der erfindungsgemäßen gepulsten Lichtquelle vereinfacht werden.

## Patentansprüche

1. Gepulste Lichtquelle (1) zum Abtragen von biologischem Gewebe,
mit Lichtmitteln (3;3a,3b) und mit einer Steuereinheit (2) zum Steuern der Lichtmittel derart, daß die Lichtmittel einerseits Ablationsimpulse (B_{A}) mit einer bestimmten Impulsfrequenz und einer zur Ablation von Gewebe ausreichenden Bestrahlungsstärke sowie andererseits eine Koagulationsstrahlung (B_{K}) mit einer zwar für eine Erwärmung von Gewebe, jedoch nicht für eine Ablation von Gewebe ausreichenden Bestrahlungsstärke erzeugen,
**dadurch gekennzeichnet,**
daß die Steuereinheit (2) die Lichtmittel (3;3a,3b) zudem derart ansteuert, daß die Lichtmittel die Koagulationsstrahlung (B_{K}) unabhängig von den Ablationsimpulsen (B_{A}) erzeugen.

2. Gepulste Lichtquelle nach Anspruch 1,
**dadurch gekennzeichnet,**
daß die Bestrahlungsstärke der Ablationsimpulse (B_{A}) im Bereich 1-250 Jcm⁻² liegt.

3. Gepulste Lichtquelle nach Anspruch 1oder 2,
**dadurch gekennzeichnet,**
daß die Impulsdauer der Ablationsimpulse (B_{A}) 100-800 µs beträgt.

4. Gepulste Lichtquelle nach einem der vorhergehenden Ansprüche,
**dadurch gekennzeichnet,**
daß die Impulsfrequenz der Ablationsimpulse (B_{A}) im Bereich 1-30 Hz liegt.

5. Gepulste Lichtquelle nach den Ansprüchen 2,3 und 4,
**dadurch gekennzeichnet,**
daß die Bestrahlungsstärke, die Impulsdauer und/oder die Impulsfrequenz der Ablationsimpulse (B_{A}) einstellbar ist.

6. Gepulste Lichtquelle nach einem der vorhergehenden Ansprüche,
**dadurch gekennzeichnet,**
daß die Bestrahlungsstärke der Koagulationsstrahlung (B_{K}) kleiner als 1 Jcm⁻² ist.

7. Gepulste Lichtquelle nach einem der vorhergehenden Ansprüche,
**dadurch gekennzeichnet,**
daß die Lichtmittel eine erste Lichtquelleneinheit (3a) zur Erzeugung der Ablationsimpulse (B_{A}) und eine zweite Lichtquelleneinheit (3b) zur Erzeugung der Koagulationsstrahlung (B_{K}) umfassen.

8. Gepulste Lichtquelle nach einem der vorhergehenden Ansprüche,
**dadurch gekennzeichnet,**
daß die Lichtmittel eine gemeinsame Lichtquelleneinheit (3) zur Erzeugung sowohl der Ablationsimpulse (B_{A}) als auch der Koagulationsstrahlung (B_{K}) umfassen.

9. Gepulste Lichtquelle nach einem der vorhergehenden Ansprüche,
**dadurch gekennzeichnet,**
daß die Ablationsstrahlung (B_{A}) Laserstrahlung mit einer Wellenlänge im infraroten Bereich ist.

10. Gepulste Lichtquelle nach einem der vorhergehenden Ansprüche,
**dadurch gekennzeichnet,**
daß die Ablationsstrahlung (B_{A}) das Licht einer gepulsten Hochdruck-Gasentladungslampe oder Laserdiode ist.

11. Gepulste Lichtquelle nach einem der vorhergehenden Ansprüche,
**dadurch gekennzeichnet,**
daß die Lichtmittel (3;3a,3b) die Koagulationsstrahlung (B_{K}) kontinuierlich erzeugen.

12. Gepulste Lichtquelle nach Anspruch 11,
**dadurch gekennzeichnet,**
daß die Bestrahlungsstärke der Koagulationsstrahlung (B_{K}) zeitlich konstant ist.

13. Gepulste Lichtquelle nach den Ansprüchen 8 und 12,
**dadurch gekennzeichnet,**
daß die gemeinsame Lichtquelleneinheit (3) ein Er : YAG-Laserkristall (10) ist, der gleichzeitig durch kontinuierliche Anregung die kontinuierliche Koagulationsstrahlung (B_{K}) und durch gepulste Anregung die Ablationsimpulse (B_{A}) erzeugt.

14. Gepulste Lichtquelle nach Anspruch 13,
**dadurch gekennzeichnet,**
daß eine Laserdiodenanordnung (8) zur axialen kontinuierlichen Anregung und eine Blitzlampe (13) zur gepulsten Anregung des Er : YAG-Laserkristalls (10) vorgesehen sind.

15. Gepulste Lichtquelle nach Anspruch 11,
**dadurch gekennzeichnet,**
daß sich die Bestrahlungsstärke der Koagulationsstrahlung (B_{K}) zeitlich verändert.

16. Gepulste Lichtquelle nach Anspruch 15,
**dadurch gekennzeichnet,**
daß sich die Bestrahlungsstärke der Koagulationsstrahlung (B_{K}) zeitlich stetig verringert.

17. Gepulste Lichtquelle nach Anspruch 15,
**dadurch gekennzeichnet,**
daß sich die Bestrahlungsstärke der Koagulationsstrahlung (B_{K}) periodisch wellenartig, zick-zackartig oder sägezahnartig verändert.

18. Gepulste Lichtquelle nach einem der Ansprüche 1-10,
**dadurch gekennzeichnet,**
daß die Lichtmittel (3;3a,3b) die Koagulationsstrahlung (B_{K}) in Form von Koagulationsimpulsen mit einer bestimmten Impulsfrequenz erzeugen.

19. Gepulste Lichtquelle nach Anspruch 18,
**dadurch gekennzeichnet,**
daß die Impulsfrequenz der Koagulationsimpulse höher ist als die Impulsfrequenz der Ablationsimpulse.

20. Gepulste Lichtquelle nach Anspruch 18 oder 19,
**dadurch gekennzeichnet,**
daß die Koagulationsimpulse (B_{K}) zumindest teilweise die Ablationsimpulse (B_{A}) überlagern.

21. Gepulste Lichtquelle nach Anspruch 6 und einem der Ansprüche 18-20,
**dadurch gekennzeichnet,**
daß die Bestrahlungsstärke, die Impulsfrequenz und/oder die Impulsdauer der Koagulationsimpulse (B_{K}) einstellbar ist.
